# EUROPÄISCHE PATENTANMELDUNG

(11) **EP 3 061 510 A1**
(43) Veröffentlichungstag der Anmeldung: **31.08.2016**
(21) Anmeldenummer: 15156901.9
(22) Anmeldetag: 27.02.2015
(51) Int. Cl.: B01D 15/18, A61K 31/35, C07D 311/80

(54) **CPC Verteilungschromatographie von Cannabinoiden**

(71) Anmelder: Bionorica Ethics GmbH, 92318 Neumarkt (DE)
(72) Erfinder: Rutz, Andreas, 91741 Dornhausen (DE)
(74) Vertreter: Simandi, Claus

(57) **Zusammenfassung**

Die Erfindung betrifft Cannabinoide und deren Isolierung und Aufreinigung als auch Gewinnung mittels *Centrifugation Partitioning Chromatography.*

## Beschreibung

Die Erfindung betrifft Cannabinode und deren Isolierung und Aufreinigung als auch Gewinnung mittels *Centrifugation Partitioning Chromatography* (kurz: CPC).

Die CPC wird zur Gewinnung und Anreicherung von Pflanzeninhaltsstoffen aus Pflanzenextrakten im analytischen, semipräparativen sowie präparativen Maßstab eingesetzt. Sie ermöglicht eine nahezu verlustfreie Separation hochkomplexer Substanzgemische aus Rohextrakten. Hersteller solcher Zentrifugal-Verteilung-Chromatographen zur Durchführung einer CPC ist z.B. Kromaton S.a.r.l (Annonay, FR).

Die CPC ist im Vergleich zur Flüssigchromatographie (HPLC) einfacher und zudem kostengünstiger, da Matrixeffekte sowie irreversible Adsorptionen an festen Phasen nicht vorkommen.

Bei der CPC Methode wird, wie bei gängigen flüssig-flüssigchromatographischen Methoden, wie zum Beispiel der High Speed Countercurrent Chromatography (HSCCC), ein 2-phasiges Lösungsmittelgemisch eingesetzt. Es kann wahlweise die obere oder die untere Phase als stationäre Phase verwendet werden. Anders jedoch als bei der HSCCC wird bei der CPC nicht mit einer Kapillarspule, sondern mit einem mit mehreren hundert Trennkammern versehenen Rotor gearbeitet. In diesen direkt hintereinander geschalteten Kammern findet die Verteilung der im Pflanzenextrakt enthaltenen Substanzen zwischen mobiler und stationärer Phase statt.

Das System wird während der Trennung in schnelle Rotation versetzt (bis zu 1.500 rpm). Dadurch wird zum einen, je nach Durchflußrichtung, die gewünschte Phase im Rotor der CPC zurückgehalten und zum anderen die Trennung der beiden Phasen durch die Zentrifugalkraft beschleunigt. Dies ermöglicht die Verwendung großer Flußgeschwindigkeiten und folglich den Durchsatz großer Substanzmengen in kurzer Zeit. Der Verteilungskoeffizient K der jeweils gewünschten Substanz zwischen den beiden Phasen sollte im Bereich zwischen 0,7 und 4,5 liegen. Bei kleinerem K eluiert die Substanz zu schnell, so dass keinerlei Trennung erfolgen kann. Bei höherem K hingegen wird die Retentionszeit für die schnelle Aufreinigung großer Mengen eines Pflanzenextrakts zu lang.

Im Stand der Technik ist beschrieben, dass Cannabinoide mittels CO₂ Extraktion aus Cannabis-Extrakten erhalten werden können (DE10051427C1). Dennoch werden die Cannabinoide, wie z.B. THC (A, "Dronabinol") oder CBD (B) nicht in absoluter Reinheit erhalten.

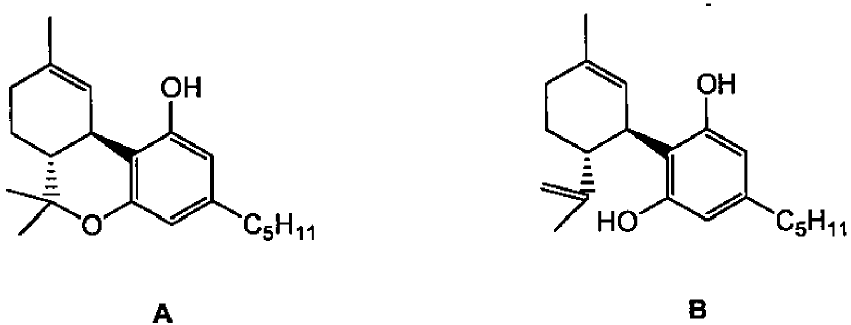

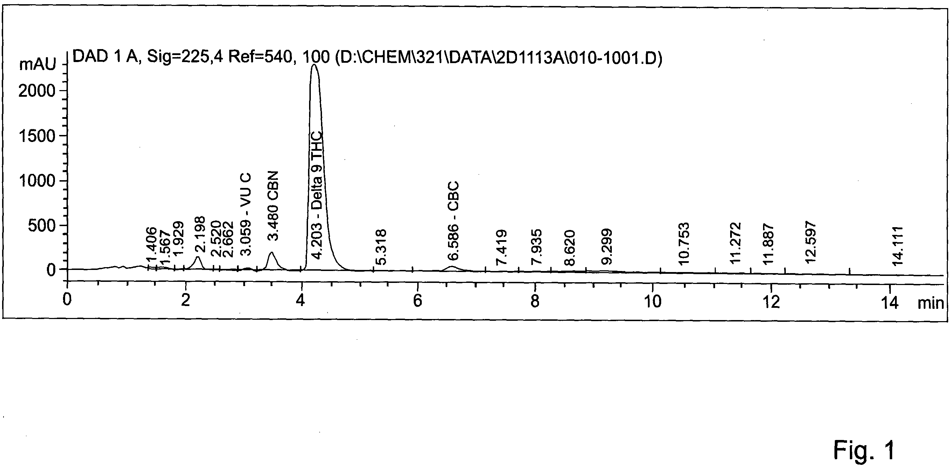

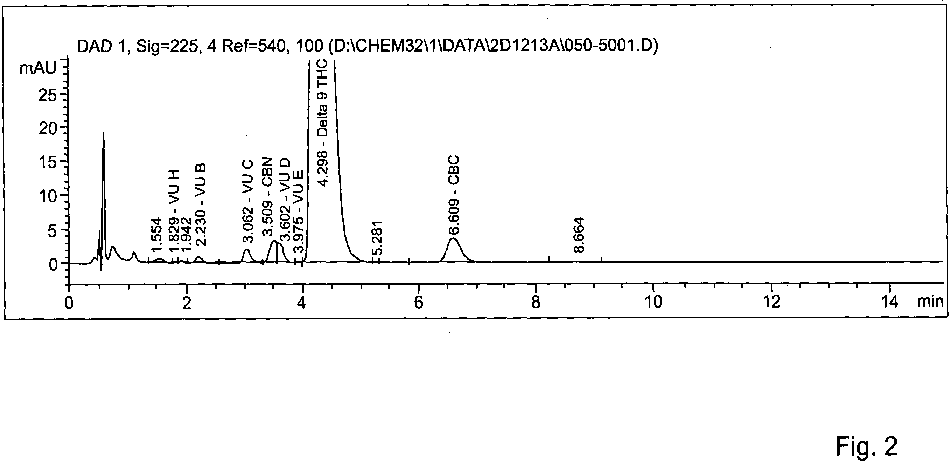

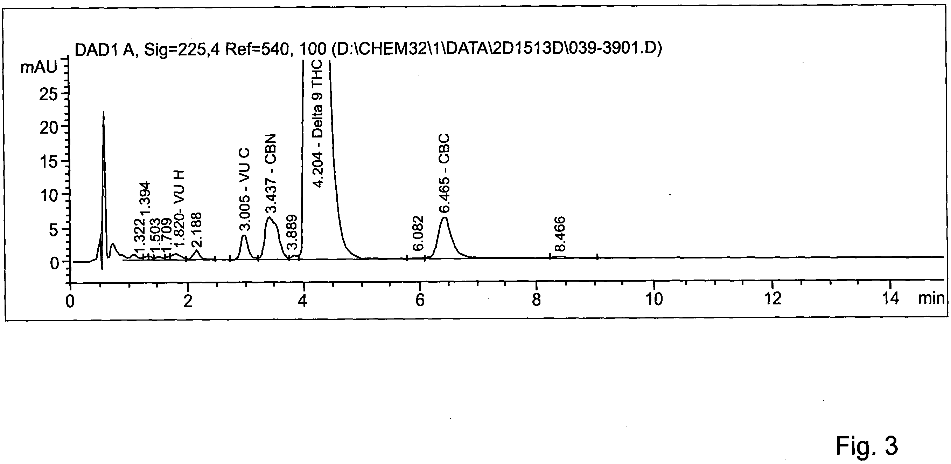

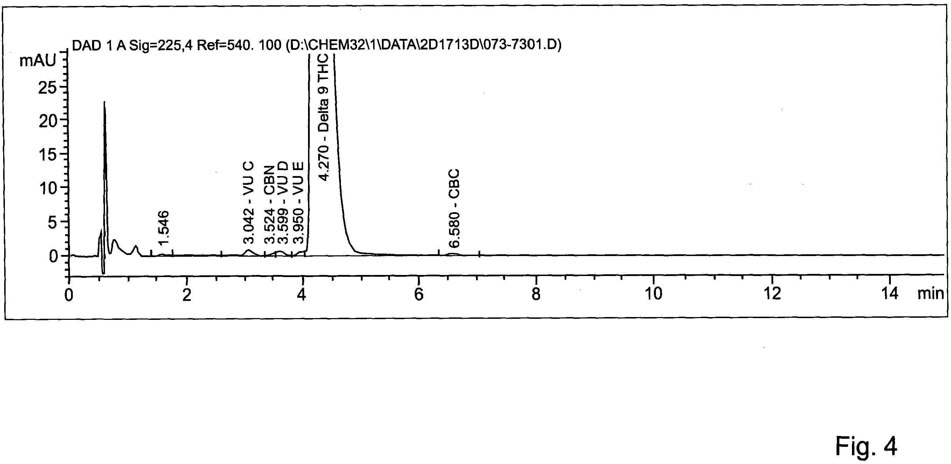

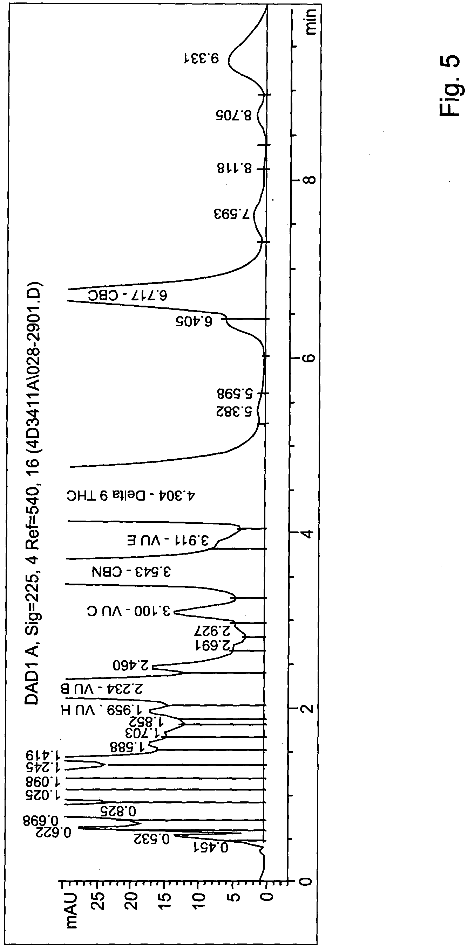

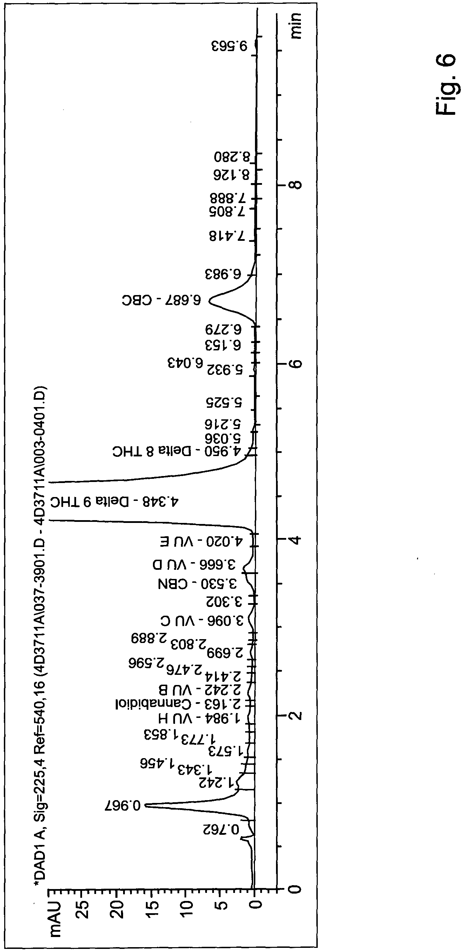

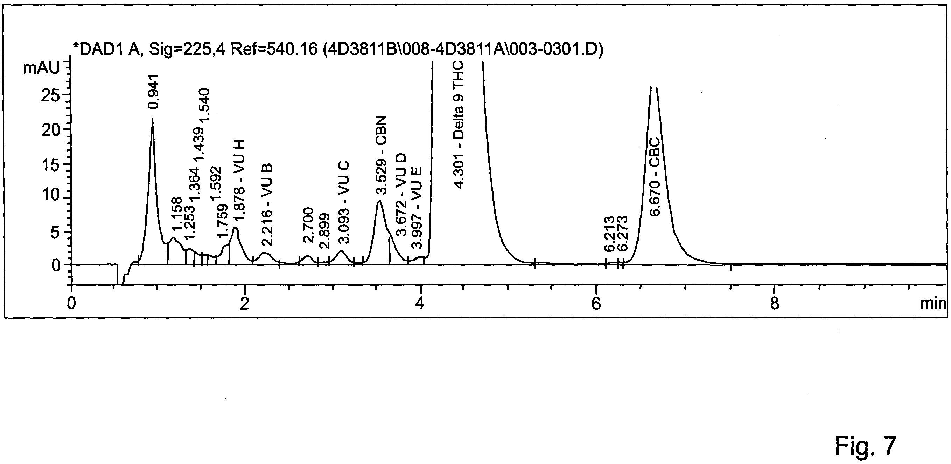

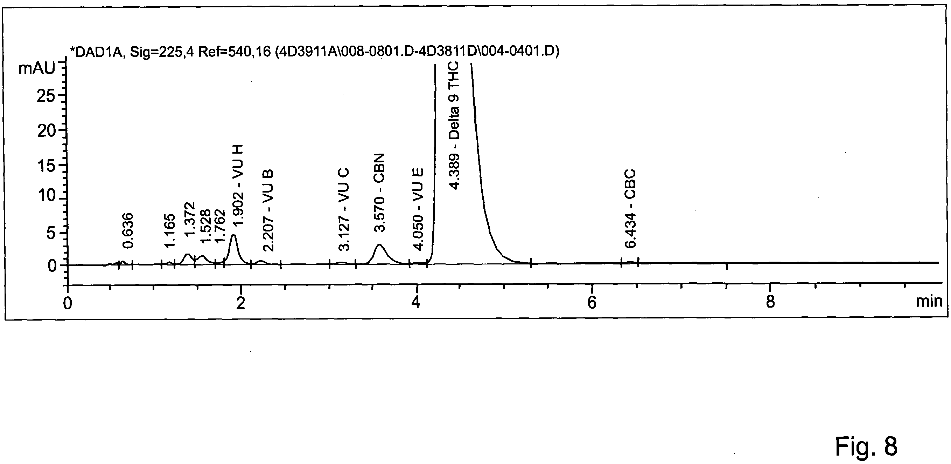

### Abb. 1: Dronabinol (A) ((6aR-trans)-6a,7,8,10a-tetrahydro-6,6,9-trimethyl-3-pentyl-6H-dibenzo[b,d]pyran-1-ol, Δ⁹-Tetrahydrocannabinol (Δ⁹THC)), Cannabidiol (CBD) (B) ((2-[1R-3-Methyl-6-(1-methylethenyl]-2-cyclohexen-1-yl]-5-pentyl-1,3-benzoldiol))

Die chromatographische Trennung und präparative Aufreinigung von Cannabinoiden stellt nach wie vor eine Herausforderung dar, insbesondere die Gewinnung von Cannabinoiden, vorzugsweise Δ⁹THC und CBD in hoher Reinheit von mehr als 95 %.

Im Stand der Technik beschreiben Hazekamp, A., 2007, Doctoral thesis, Leiden University und Arno Hazekamp, Ruud Simons, Anja Peltenburg-Looman, Melvin Sengers, Rianne van Zweden, Robert Verpoorte, Preparative isolation of cannabinoids from Cannabis sativa by centrifugal partition chromatography, J. Liq. Chrom. Rel. Technol. 2004, 27(15): 2421-2439, die präparative Gewinnung von Cannabinoiden mittels CPC. Allerdings wird im Stand der Technik ein Zwei-Phasen System auf der Basis von Hexan verwendet und Ausbeuten von max. 95 % erreicht.

Es stellt sich somit die Aufgabe, ein verbessertes Verfahren für die Trennung und/oder Reinigung von Cannabinoiden aus Cannabis Pflanzenextrakten bereitzustellen, welches die vorliegenden Nachteile zumindest teilweise beseitigt und insbesondere in der Lage ist, bei vielen Anwendungen eine höhere Ausbeute und Reinheit an Cannabinoiden, insbesondere CBD und / oder THC präparativ bereitzustellen.

Die Aufgabe wird durch ein Verfahren nach einem der Patentansprüche gelöst.

Demgemäß wird ein Verfahren zur Trennung und/oder Reinigung von Cannabinoiden bereitgestellt, enthaltend mindestens einen Flüssig-flüssig-Verteilungschromatographieschritt, bzw. die Verwendung eines Zentrifugal-Verteilung Chromatographen zur Flüssig-flüssig-Verteilungschromatographie zur Trennung und/oder Reinigung von Cannabinoiden, wobei ein Lösungsmittel aus Heptan, n-Heptan, ausgewählt ist, das durch Zentrifugalkraft stationär gehalten wird und eine zweite nicht mischbare flüssige Phase.

Das erfindungsgemäße Verfahren und/oder die erfindungsgemäße Verwendung zeigen vorteilhaft einen geringen Eluentenverbrauch neben keinen Ablagerungen an einer stationären Phase, so dass keine Regeneration der stationären Phase, bzw. Aufbereitung oder Entfernung von störenden Komponenten vonnöten ist. Die Produktwiederfindung ist nahezu quantitativ, da stationäre und mobile Phase einfach vertauscht werden können. Zudem wird die stationäre Phase bei jedem Lauf vollständig erneuert und kann durch Destillation einfach aufgereinigt bzw. erneuert werden. Die Reinheit der isolierten Cannabinoide ist oftmals so hoch, dass weitere chromatographische Aufreinigungsschritte entfallen können. Das Verfahren ist einfach ebenfalls im großtechnischen Maßstab durchführbar. Die Ausbeute an Cannabinoiden ist gegenüber dem Stand der Technik oftmals stark erhöht, wie im Folgenden noch genauer ausgeführt wird. Die Anzahl an benötigten Prozessstufen kann gegenüber alternativen Techniken meist deutlich vermindert werden. Besonders vorteilhaft, kann die Gewinnung von Cannabinoiden aus beliebigen Cannabispflanzen und deren Extrakte erfolgen.

Erfindungsgemäß ist für Cannabinoide die vorteilhafte Verwendung der beiden nicht miteinander mischbaren Lösungsmittel Acetonitril und Heptan bei einem Fluß von 50 bis 600 mL pro min. vorgesehen, vorzugsweise bei einem Fluss von 200 bis 300 mL pro min. während der Trennung und max. Fluss beim Spülen, bei einer Umdrehungszahl von 50 bis 1.500 rpm, vorzugsweise einer Umdrehungszahl von 900 bis 1.100 rpm während der Trennung. Weiterhin ist bevorzugt die Verwendung der oberen Phase als stationäre Phase.

In einer weiteren bevorzugten Ausführungsform kann dem jeweiligen Lösungsmittel t-Butylmethylether (TBME) zugesetzt werden und zwar 1 bis 15 % (v/v), vorzugsweise 9 bis 13 % (v/v).

Die zweite nicht mischbare flüssige Phase kann auch solche Lösungsmittel umfassen, wie Methanol, Ethylacetat oder Wasser, welche ggfs. mit 1-15 % t-Butylmethylether (TBME) versetzt.

Erfindungsgemäße Beispiele sind:
n-Heptan/ Acetonitril 1:1 (ohne t-Butylmethylether); n-Heptan/Ethylacetat/Acetonitril; n-Heptan/Ethylacetat/t-Butylmethylether/ Acetonitril; n-Heptan/ Ethylacetat/Methanol/Wasser.

Jedoch ist erfindungsgemäß Actetonitril, ggfs. versetzt mit 1-15 % t-Butylmethylether (TBME) besonders bevorzugt.

Weiterhin kann vorgesehen sein, dass das erfindungsgemäße Verfahren ein oder mehrfach durchlaufen wird (siehe z.B. Beispiel 1).

Die vorgenommene Auswahl dieser Lösungsmittel erlaubt eine Gewinnung von Cannabinoiden, vorzugsweise Δ⁹THC und CBD in hoher Reinheit von mehr als 95 %.

Daher betrifft die Erfindung einen Cannabis-Extrakt erhältlich oder erhalten nach dem erfindungsgemäßen Verfahren, wobei die Dronabinol Reinheit mehr als 95 %, insbesondere 99,6 % beträgt.

Bei Verwendung eines 12.500 mL fassenden CPC-Rotors ist zudem vorteilhaft eine Aufreinigung von bis zu 100 g Extrakt pro Lauf möglich. Pro Trennung werden inklusive Vorbereitungs- und Spülzeiten vorteilhaft lediglich 120 bis 160 min benötigt. Die Cannabinoide enthaltenden Fraktionen werden von den organischen Lösungsmitteln befreit. Nach Einengen im Vakuum erhält man Extrakte mit einem Massengehalt an gewünschtem Cannabinoid von bis zu 70-99 %, vorzugsweise mehr als 95 %.

Im Rahmen dieser Erfindung, werden unter Cannabinoiden insbesondere die folgenden Stoffe verstanden:
*Cannabigerol-artige (CBG) :* Cannabigerol ((E)-CBG-C₅), Cannabigerol Monomethylether ((E)-CBGM-C₅ A), Cannabinerolsäure A ((Z)-CBGA-C₅ A), Cannabigerovarin ((E)-CBGV-C₃, Cannabigerolsäure A ((E)-CBGA-C₅ A), Cannabigerolsäure A Monomethylether ((E)-CBGAM-C₅ A), Cannabigerovarinsäure A ((E)-CBGVA-C₃ A);
*Cannabichromen-artige (CBC) :* Cannabichromen (CBC-C₅), Cannabichromensäure A (CBCA-C₅ A), Cannabichromevarin (CBCV-C₃), Cannabichromevarinsäure A (CBCVA-C3 A);
*Cannabidiol-artige (CBD):* Cannabidiol (CBD-C₅), Cannabidiol Monomethylether (CBDM-C₅), Cannabidiol-C4 (CBD-C₄), Cannabidivarin (CBDV-C₃), Cannabidiorcol (CBD-C₁), Cannabidiolsäure (CBDA-C₅), Cannabidivarinsäure (CBDVA-C₃);
*Cannabinodiol-artige (CBND) :* Cannabinodiol (CBND-C₅), Cannabinodivarin (CBND-C₃);
*Tetrahydrocannabinol-artige (THC):* Δ9-Tetrahydrocannabinol (Δ9-THC-C₅), Δ9-Tetrahydrocannabinol-C4 (Δ9-THC-C₄), Δ9-Tetrahydrocannabivarin (Δ9-THCV-C₃), Δ9-Tetrahydrocannabiorcol (Δ9-THCO-C₁), Δ9-Tetrahydrocannabinolsäure (Δ9-THCA-C₅ A), Δ9-Tetrahydrocannabinolsäure B (Δ9-THCA-C₅ B), Δ9-Tetrahydrocannabinolsäure-C4 (Δ9-THCA-C₄ A und/oder B), Δ9-Tetrahydrocannabivarinsäure A (Δ9-THCVA-C₃ A), Δ9-Tetrahydrocannabiorcolsäure (Δ9-THCOA-C₁ A und/oder B), (-)-Δ8-trans-(6aR,10aR)-Δ8-Tetrahydrocannabinol (Δ8-THC-C₅),(-)-Δ8-trans-(6aR,10aR)-Tetrahydrocannabinolsäure A (Δ8-THCA-C₅ A); (-)-(6aS,10aR)-Δ9-Tetrahydrocannabinol ((-)-cis-Δ9-THC-C5);
*Cannabinol-artige (CBN):* Cannabinol CBN-C₅, Cannabinol-C4 (CBN-C₄), Cannabivarin (CBN-C₃), Cannabinol-C2 (CBN-C₂), Cannabiorcol (CBN-C₁), Cannabinolsäure A (CBNA-C₅ A), Cannabinolmethylether (CBNM-C₅)
*Cannabitriol-artige (CBT):* (-) - (9R,10R)-trans-Cannabitriol ( (-) -trans-CBT-C₅), (+)-(9S,10S)-Cannabitriol ((+) -trans-CBT-C₅), (±)-(9R,10S/9S,10R)-Cannabitriol ((±) -cis-CBT-C₅), (-)-(9R,10R)-trans[10-O-Ethyl-cannabitriol] ((-)-trans-CBT-OEt-C₅), (±)-(9R,10R/9S,10S)-Cannabitriol-C3 ((±)-trans-CBT-C₃),8,9-Dihydroxy-Δ6a(10a) tetrahydrocannabinol (8,9-Di-OH-CBT-C₅), Cannabidiolsäure A (CBDA-C₅ 9-OH-CBT-C5 ester), (-)-(6aR,9S,10S,10aR)-9,10-Dihydroxy-hexahydrocannabinol, Cannabiripsol Cannabiripsol-C5, (-)-6a,7,10a-Trihydroxy-Δ9-tetrahydrocannabinol ((-)-Cannabitetrol), 10-Oxo-Δ6a(10a) tetrahydrocannabinol (OTHC);
*Cannabielsoin-artige (CBE) :* (5aS, 6S, 9R, 9aR) - C₅-Cannabielsoin (CBE-C₅), (5aS,6S,9R,9aR)-C₃-Cannabielsoin (CBE-C₃), (5aS,6S,9R,9aR)-Cannabielsoinsäure A (CBEA-C₅ A), (5aS, 6S, 9R, 9aR) -Cannabielsoinsäure B (CBEA-C₅ B), (5aS, 6S, 9R, 9aR) -C3-Cannabielsoinsäure B (CBEA-C₃ B), Cannabiglendol-C3 (OH-iso-HHCV-C₃), Dehydrocannabifuran (DCBF-C₅), Cannabifuran (CBF-C₅) ;
*Isocannabinoide:* (-)-Δ7-trans- (1R, 3R, 6R) - Isotetrahydrocannabinol, (±)-Δ7-1,2-cis-(1R,3R,6S/1S,3S,6R)- Isotetrahydrocannabivarin, (-)-Δ7-trans-(1R,3R,6R)- Isotetrahydrocannabivarin;
*Cannabicyclol-artige (CBL) :* (±) - (1aS, 3aR, 8bR, 8cR) - Cannabicyclol (CBL-C₅), (±) - (1aS, 3aR, 8bR, 8cR) - Cannabicyclolsäure A (CBLA-C₅ A), (±) - (1aS, 3aR, 8bR, 8cR) - Cannabicyclovarin (CBLV-C₃);
*Cannabicitran-artige (CBT):* Cannabicitran (CBT-C₅) ;
*Cannabichromanon-artige (CBCN) :* Cannabichromanon (CBCN-C₅), Cannabichromanon-C3 (CBCN-C₃), Cannabicoumaronon (CBCON-C₅).

Besonders bevorzugt sind jedoch Cannabidiol (CBD-C₅) und Δ9-Tetrahydrocannabinol (Δ9-THC-C₅).

Unter dem Begriff "Flüssig-flüssig-Verteilungschromatographieschritt" im Sinne der vorliegenden Erfindung wird insbesondere eine Chromatographie verstanden, bei der wie folgt vorgegangen wird:

Eine bestimmte Menge eines Substanzgemisches wird mit einer flüssigen mobilen Phase durch eine stationär gehaltene Phase geleitet, wobei diese durch eine Zentrifugalkraft stationär gehalten wird. In einer weiteren bevorzugten Ausführungsform kann die Flüssig-flüssig-Verteilungschromatographie kontinuierlich durchgeführt werden. Hierzu werden die beiden Phasen im Gegenstrom geführt und eine kontinuierliche Abtrennung statt eines zeitversetzten (diskontinuierlichen) Austritts wird erreicht (siehe z.B. Yin, Lianhong; Li, Yingnan; Lu, Binan; Jia, Yujie; Peng, Jinyong, Trends in Counter-Current Chromatography: Applications to Natural Products Purification Separation and Purification Reviews (2010), 39(1-2), 33-62).

Entsprechend ihrer unterschiedlich starken Wechselwirkungen mit der stationären Phase treten die Substanzen kontinuierlich oder diskontinuierlich aus und können getrennt werden. Während jedoch bei der Flüssigchromatographie die stationäre Phase aus einem gepackten Festbett in einer Säule besteht, handelt es sich bei der Flüssig-flüssig-Verteilungschromatographie um eine zweite nicht mischbare flüssige Phase, die durch geeignete Vorrichtungen, wie z. B. einen Rotor durch Zentrifugalkraft stationär gehalten wird, insbesondere mittels einem entsprechenden Zentrifugal-Verteilung Chromatograph (supra).

"Cannabis Extrakt" im Sinne dieser Erfindung bedeutet ein beliebig aufgearbeiteter Extrakt aus einer Cannabispflanze oder Hanfpflanze, welche Cannabinoide enthält. Der Extrakt kann ein Primärextrakt, oder teilaufgearbeiteter Extrakt sein. Die Herstellung von Cannabis Extrakten ist im Stand der Technik hinreichend beschrieben. Geeignete Cannabispflanzen oder (Faser)hanfpflanzen sind solche wie Drogenhanf, Faserhanf.

In einer weiteren bevorzugten Ausführungsform kann mindestens eine präparative Säule (Festphase, wie z.B. Kieselgel) vor- oder nachgeschaltet sein (Beispiel 2).

### Beispiele und Figuren:

Diese Beispiele und Figuren dienen ausschließlich zur Erläuterung der Erfindung, ohne die Erfindung auf diese Beispiele zu beschränken.

### Beispiel 1: THC aus Industriehanf

Gewinnung von THC (Dronabinol) aus Drogenhanf: Extraktion Cannabis flos mittels Heptan, Decarboxylierung bei 120°C im Vakuum, Auflösen in Heptan, Aufreinigung in der CPC.

### 1. CPC

### 1. Lauf: quantitative Abtrennung CBN, anteilige Abtrennung CBC:

Fließmittel: Acetonitril TBME ca. 12%
Stationäre Phase: Heptan TBME ca. 10%
aus Dronabinol 84,1% mit ca. 15,9% Verunreinigungen, wobei 5,4% CBN und 2,2% CBC sind.
Farbe: tiefbraun bis schwärzlich.

### Figur 1

Es entsteht:
Dronabinol 97,7% mit ca. 2,3% Verunreinigungen, wobei 0,4% CBN und 0,9% CBC sind.
Farbe: gelb.

### Figur 2

Optional 2. Lauf: quantitatve Abreinigung von CBC
Fließmittel: Acetonitril TBME ca. 12%
Stationäre Phase: Heptan TBME ca. 10%
aus Dronabinol 97,7% mit ca. 2,3% Verunreinigungen, wobei 0,8% CBN und 0,9% CBC sind.
Farbe: gelb.

### Figur 3

Es entsteht:
Dronabinol 99,6% mit ca. 0,4 % Verunreinigungen, wobei 0,04% CBN und 0,05% CBC sind.
Farbe: gelblich (weitere Farbaufhellung)

### Figur 4

Beispiel 2: CPC mit nachgelagertem Lauf über Kieselgelsäule
Lauf: quantitative Abtrennung CBN
keine Abtrennung CBC
Fließmittel: Acetonitril purum
Stationäre Phase: Heptan purum
aus Dronabinol 81% mit ca. 19% Verunreinigungen, wobei 4,2% CBN und 1,6% CBC sind.
Farbe schwärzlich bis tiefbraun

### Figur 5

Es entsteht:
Dronabinol 94,5% mit ca. 5,5% Verunreinigungen, wobei 0,16% CBN und 1,4% CBC sind.
Farbe: gelb.

### Figur 6

Anschließend präparative Chromatographie über Kieselgel zur quantitativen Abtrennung von CBC:
Fließmittel Heptan 3% TBME
aus Dronabinol 96,4% mit ca. 3,6% Verunreinigungen, wobei 0,36% CBN und 1,8% CBC sind.
Farbe: gelb.

### Figur 7

Es entsteht:
Dronabinol 98,6% mit ca. 1,4% Verunreinigungen, wobei 0,4% CBN und 0,0% CBC sind.
Farbe: farblos.

*Figur 8*

## Patentansprüche

1. Verfahren zur Trennung und/oder Reinigung von Cannabinoiden aus einem Cannabis Extrakt, enthaltend mindestens einen Flüssig-flüssig-Verteilungschromatographieschritt, wobei ein Lösungsmittel aus Heptan ausgewählt ist, das durch Zentrifugalkraft stationär gehalten wird und eine zweite nicht mischbare flüssige Phase.

2. Verfahren zur Trennung und/oder Reinigung von Cannabinoiden aus einem Cannabis Extrakt nach Anspruch 1, wobei die zweite Phase Acetonitril, Methanol, Ethylacetat oder Wasser ist.

3. Verfahren zur Trennung und/oder Reinigung von Cannabinoiden aus einem Cannabis Extrakt nach Anspruch 1 oder Anspruch 2, wobei Heptan und/oder die zweite nicht mischbare flüssige Phase mit 1 bis 15 % (v/v), 9 bis 13 % (v/v) t-Butlymethylether versetzt ist.

4. Verfahren zur Trennung und/oder Reinigung von Cannabinoiden aus einem Cannabis Extrakt nach einem der vorstehenden Ansprüche, mit einem Fluss von 50 bis 600 mL pro min. und/oder einer Umdrehungszahl von 50 bis 1.500 rpm.

5. Verfahren zur Trennung und/oder Reinigung von Cannabinoiden aus einem Cannabis Extrakt nach einem der vorstehenden Ansprüche, wobei die beiden Phasen im Gegenstrom geführt werden und eine kontinuierliche Abtrennung erfolgt.

6. Verfahren zur Trennung und/oder Reinigung von Cannabinoiden aus einem Cannabis Extrakt nach einem der vorstehenden Ansprüche, wobei mindestens eine präparative Säule vor- oder nachgeschaltet ist.

7. Cannabis-Extrakt erhältlich nach einem Verfahren der vorstehenden Ansprüche, **dadurch gekennzeichnet, dass** die Dronabinol Reinheit mehr als 95 %, insbesondere 99,6 % beträgt.

8. Verwendung eines Zentrifugal-Verteilung Chromatograph zur Trennung und/oder Reinigung von Cannabinoiden nach einem Verfahren nach einem der vorstehenden Patentansprüche.
